# EUROPEAN PATENT SPECIFICATION

(11) **EP 1 212 056 B1**
(45) Date of publication and mention of the grant of the patent: **07.12.2005**
(21) Application number: 00952645.0
(22) Date of filing: 08.08.2000
(51) Int. Cl.: A61K 31/40, A61P 25/00

(54) **METHODS AND COMPOSITIONS FOR BISUBSTRATE INHIBITORS OF ACETYLTRANSFERASES**
METHODEN UND ZUSAMMENSETZUNGEN ZUR BILDUNG VON BISUBSTRATINHIBITOREN VON ACETYLTRANSFERASEN
TECHNIQUES ET COMPOSITIONS POUR INHIBITEURS D'ACETYLTRANSFERASES A DEUX SUBSTRATS

(30) Priority: 13.08.1999 US 374742
(43) Date of publication of application: 12.06.2002
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852-3804 (US)
(72) Inventor: KLEIN, David, C., Rockville, MD 20852 (US); NAMBOODIRI, M., A., A., Gaithersburg, MD 20878 (US); WELLER, Joan, L., Darnestown, MD 20874 (US); KOWALAK, Jeffrey, A., Alexandria, VA 22307 (US); HO, Anthony, K., Edmonton, Alberta T6R 2E6 (CA)
(74) Representative: Leissler-Gerstl, Gabriele
(86) International application number: PCT/US2000/021631
(87) International publication number: WO 2001/012185

(56) References cited:
- NAMBOODIRI M.A.A., ET AL.: "Rapid Nocturnal Increase in Ovine Pineal N-Acetyltransferase Activity and Melatonin Synthesis: Effects of Cycloheximide" JOURNAL OF NEUROCHEMISTRY, vol. 45, no. 3, 1985, pages 832-835, XP000944520
- DE ANGELIS, J., ET AL.: "Kinetic Analysis of the Catalytic Mechanism of Serotonin N-Acetyltransferase (EC 2.3.1.87)" JOURNAL OF BIOLOGICAL CHEMISTRY, vol. 273, no. 5, 30 January 1998 (1998-01-30), pages 3045-3050, XP000941652

## Description

### BACKGROUND OF THE INVENTION

### Field of the Invention

The present invention is directed to the formation and action of a bisubstrate inhibitor of an acetyltransferase. In particular, the invention relates to contacting acetyl coenzyme A (AcCoA) with an alkylating derivative of an acetyl acceptor substrate in the presence of an acetyltransferase specific for that substrate to form a bisubstrate inhibitor with inhibitory action on that acetyltransferase. This bisubstrate inhibitor can be employed in a variety of biological settings to modulate the activity of specific acetyltransferases.

### Background Art

One of the most common transformations in biology is acetylation. Acetyltransferase enzymes act by binding the universal acetyl group donor, acetyl coenzyme A (AcCoA), and affecting transfer of the acetyl group to a specific acceptor substrate, yielding CoA and the corresponding acetylated compound. In contrast to the AcCoA donor, acetyl acceptors are diverse and highly varied, ranging in size from diamines to proteins. Specificity of acetyl transfer is determined by the highly selective and specific "lock and key" binding of narrow groups of acetyl acceptors to correspondingly specific acetyltransferase enzymes. Each of these enzymes has a different and important role in biology.

An example of an important acetyltransferase is provided by serotonin N-acetyltransferase (arylalkylamine N-acetyltransferase, AANAT, E.C. 2.3.1.87), which binds AcCoA and a narrow set of arylalkylamines including serotonin, tryptamine, and phenylethylamine, and releases CoA and the corresponding N-acetyl-arylalkylamine, i.e. N-acetylserotonin, N-acetyltryptamine and N-acetylphenylethylamine (1,2) . This enzyme is of biological importance because it is involved in a broad range of biological processes through the key role it plays in regulating the synthesis of melatonin (N-acetyl 5-methoxytryptamine)(3).

Specific inhibitors of enzymes are of great value as tools and drugs in medicine and agriculture. Development of such drugs is a common goal with great commercial and practical interest. Efforts to develop inhibitors of acetyltransferases have involved the *in vitro* chemical synthesis of bisubstrate inhibitors, which are compounds that share characteristics of CoA and of the specific acetyl group acceptors. A highly potent bisubstrate inhibitor of AANAT is CoA-*S*-*N*-acetyltryptamine, which binds to the catalytic pocket of the enzyme (4,5). A problem with this type of inhibitor is that CoA is expensive, difficult to synthesize and does not pass through the cell membrane because it is charged. Accordingly, it is impractical to use CoA compounds as drugs.

The present invention overcomes previous shortcomings in this art by providing a method of producing a bisubstrate inhibitor within a cell, allowing for the selective inhibition of a specific acetyltransferase at the site of its action. The methods of this invention can be used in a variety of treatment protocols in which intracellular control of the activity of a particular acetyltransferase is desired.

### SUMMARY OF THE INVENTION

The present invention provides an ex-vivo method of producing a bisubstrate inhibitor in a cell, comprising introducing into the cell an alkylating derivative of an acetyl acceptor substrate for an acetyltransferase present in the cell.

Further provided is an ex vivo method of inhibiting the activity of an acetyltransferase in a cell, comprising introducing into the cell an alkylating derivative of an acetyl acceptor substrate for an acetyltransferase present in the cell under conditions whereby a bisubstrate inhibitor will be produced, thereby inhibiting the activity of the acetyltransferase in the cell.

The subject matter of the present invention is defined in the claims and concerns ex vivo methods as defined above and beyond. Moreover, the present invention provides alkylating derivatives of an acetyl acceptor substrate for an acetyltransferase for use in medical treatment and particularly for inhibiting the activity of an acetyltransferase in a cell, for inhibiting melatonin production and for increasing the amount of serotonin.

In addition, the present invention provides a method of inhibiting melatonin production in a cell which produces melatonin, comprising introducing into the cell an alkylating derivative of the acetyl acceptor substrate of AANAT, which can be N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine.

A method of increasing the amount of serotonin in a cell which produces serotonin is also provided herein, comprising introducing into the cell an alkylating derivative of the acetyl acceptor substrate of AANAT, which can be N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine.

In a further embodiment, the present invention provides the use of an alkylating derivative of the acetyl acceptor substrate of AANAT, which can be N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine. for preparing a medicament for treating a disorder in a subject caused by a decreased amount of serotonin.

Also provided herein is a cell comprising a bisubstrate inhibitor, wherein the bisubstrate inhibitor comprises an alkylating derivative of an acetyl acceptor substrate for an acetyltransferase present in the cell and CoA.

Various other objectives and advantages of the present invention will become apparent from the following detailed description.

### BRIEF DESCRIPTION OF THE DRAWINGS

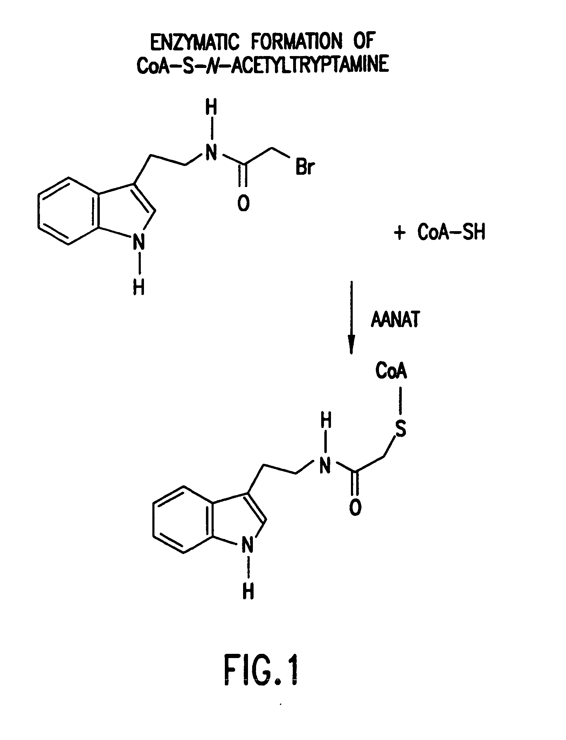

Figure 1 shows the AANAT-dependent catalytic formation of CoA-*S*-*N*-acetyltryptamine from CoA and *N*-acetyltryptamine.

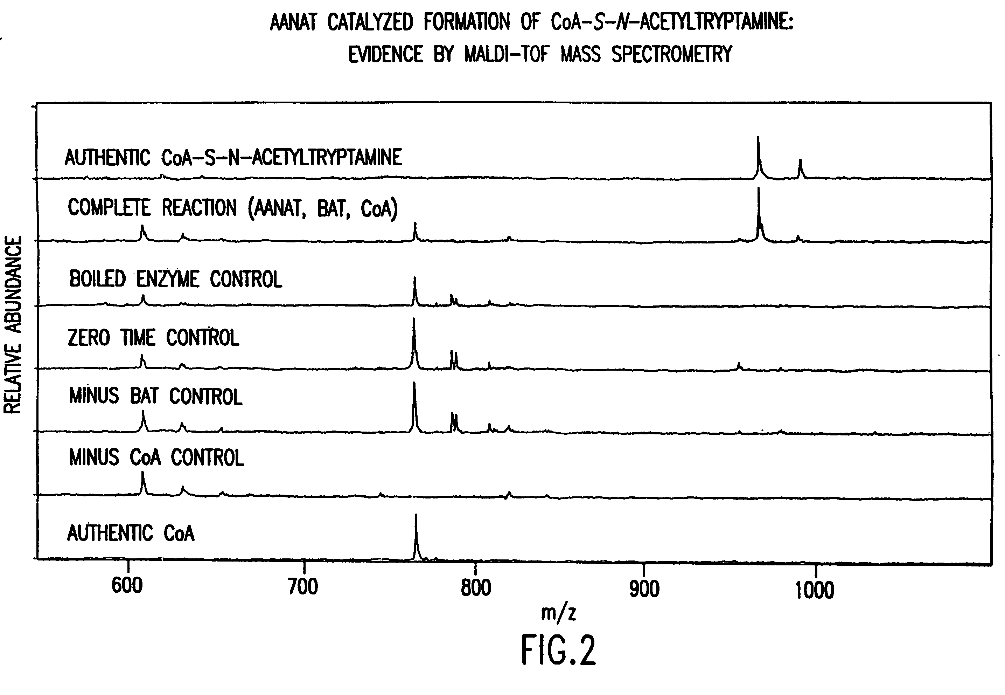

Figure 2 provides MALDI-TOF-MS evidence of AANAT catalyzed synthesis of Co A *S*-*N*-acetyltryptamine. Details of the AANAT incubation in the presence of CoA (20 µM) and N-bromoacetyltryptamine (20 µM) are described herein. Where AANAT was present, the amount of activity per tube represents 10 µmoles of product formed per hour. Samples of chloroform-extracted aqueous samples were used for MALDI-TOF analysis, as described herein. The amount of authentic CoA-*S*-*N*-acetyltryptamine was ~ 10 picomoles.

### DETAILED DESCRIPTION OF THE INVENTION

As used herein, "a" can include multiples. For example, "a" cell can mean a single cell or many cells.

The present invention is based on the surprising discovery that a bisubstrate inhibitor which is specific for a particular acetyltransferase can be formed in a cell in which the particular acetyltransferase and AcCoA are present by introducing into the cell an alkylating derivative of an acetyl acceptor substrate which is specific for the acetyltransferase to be inhibited. Formation of the bisubstrate inhibitor in the presence of the acetyltransferase within the cell occurs at very low concentrations in a highly efficient manner because the enzyme binds and positions the reactants in the most favorable position to promote formation of the bisubstrate inhibitor.

Thus, the present invention provides an ex-vivo method of producing a bisubstrate inhibitor in a cell, comprising introducing into the cell an alkylating derivative of an acetyl acceptor substrate for an acetyltransferase present in the cell.

The particular acetyltransferase to be targeted for inhibition can be naturally produced by the cell or it can be produced in the cell as a result of the introduction into the cell of a nucleic acid which encodes the acetyltransferase of interest. The nucleic acid can be introduced into the cell by any of a variety of mechanisms for introducing exogenous nucleic acid into a cell for expression to yield a specific gene product. Such methods are well known in the art, as described in further detail herein and can include transduction, transfection and/or transformation of the cell as these terms are commonly known in the art.

The present invention additionally provides an ex-vivo method of inhibiting the activity of an acetyltransferase in a cell, comprising introducing into the cell an alkylating derivative of an acetyl acceptor substrate for an acetyltransferase present in the cell under conditions whereby a bisubstrate inhibitor will be produced, thereby inhibiting the activity of the acetyltransferase in the cell. As described herein, the acetyltransferase of this method can be naturally produced by the cell or the acetyltransferase can be present in the cell as a result of the expression in the cell of an exogenous nucleic acid encoding the acetyltransferase.

The alkylating derivative of the acetyl acceptor substrate for the present invention can be a N-bromoacetylated acetyl acceptor substrate, a N-chloroacetylated acetyl acceptor substrate and/or a N-fluoroacetylated acetyl acceptor substrate. Furthermore, more than one alkylating derivative of an acetyl acceptor substrate can be introduced into a cell or administered to a subject of this invention, in any combination of the alkylating derivatives of an acetyl acceptor substrate of this invention. For example, two different N-bromoacetylated acetyl acceptor substrates can be administered to a cell and/or to a subject, or a N-bromoacetylated acetyl acceptor substrate and a N-chloroacetylated acetyl acceptor substrate can be administered to a cell and/or to a subject of the present invention.

As an example, the acetyltransferase of this invention can be, any of the acetyltransferases listed in Table 1 and the alkylating derivative of the acetyl acceptor substrate (derivative substrate) of this invention can be, any of the N-bromoacetylated substrate derivatives in Table 1, listed opposite the respective acetyltransferase upon which it can act in an inhibitory manner. However, it is to be understood that the bromoacetyl group of any of the substrate derivatives in Table 1 can be substituted for a chloroacetyl group or a fluoroacetyl group to produce a substrate derivative of this invention.

As a more specific example, when the acetyltransferase of the present invention is serotonin N-acetyltransferase (arylalkylamine N-acetyltransferase (AANAT, E.C. 2.3.1.87)), which regulates the synthesis of melatonin, the alkylating derivative of the acetyl acceptor substrate can be N-bromoacetyltryptamine, N-bromoacetylserotonin, N-bcomoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine, as well as any other alkylating derivative of an acetyl acceptor substrate for AANAT that is now known or later identified.

Thus, the present invention further provides an ex-vivo method of inhibiting melatonin production in a cell which produces melatonin, comprising introducing into the cell an alkylating derivative of the acetyl acceptor substrate of AANAT which can be, N-bromoacetyltryptamine, N-bromoacetylserotonin, N-bromoacetylphenylethylamine, N-bromo-acetyl-methoxytryptamine, N-bromoacetyltyramine, N-chioroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine, as well as any other alkylating derivative of an acetyl acceptor substrate for AANAT that is now known or later identified. That melatonin production has been inhibited in the cell by the method of this invention can be determined according to assays well known in the art for measuring melatonin production.

In addition, the present invention provides an ex-vivo method of increasing the amount of serotonin in a cell which produces serotonin, comprising introducing into the cell an alkylating derivative of the acetyl acceptor substrate of AANAT which can be N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine, as well as any other alkylating derivative of an acetyl acceptor substrate for AANAT that is now known or later identified. That the amount of serotonin in the cell has been increased by the method of this invention can be determined according to assays well known in the art for measuring an amount of serotonin.

The present invention also contemplates the use of an alkylating derivative as defined above for preparing a drug for treating a disorder caused by a decreased amount of serotonin, comprising administering to the subject an alkylating derivative of the acetyl acceptor substrate of AANAT which can be, N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine, as well as any other alkylating derivative of an acetyl acceptor substrate for AANAT that is now known or later identified.

The disorder caused by a decreased amount of serotonin can be depression, obsessive compulsive disorder, schizophrenia, mania, sleep/wake disorder, panic attack, migraine headache, cluster headache, insomnia, bipolar disease and/or attention disorder, as well as any other disorder now known or later identified to be caused by a decreased amount of serotonin in a subject.

The subject of this invention can be any animal which produces any of the acetyltransferases of this invention naturally or any animal which can produce any of the acetyltransferases of this invention by expression of exogenous nucleic acid encoding an acetyltransferase of this invention. The subject of this invention is preferably a mammal and is most preferably a human.

It would be well understood by one of skill in the art that the substrate derivatives of this invention can be modified according to protocols well known in the art to promote optimal binding and formation of a bisubstrate inhibitor in a cell without nonspecific alkylation. Such modified substrate derivatives can be tested *in vivo* according to standard methods to allow for identification of those substrate derivatives with optimal activity and efficacy.

For example, a substrate derivative of this invention can be administered to a subject and the intended specific effect of the drug in the subject can be monitored, along with general indices of metabolism as an indication of nonspecific effects. From these results, compounds can be identified which have the strongest intended effect relative to non-specific effects. As a specific example, in the case of arylalkylamine N-acetyltransferase, melatonin production by pineal cells can be monitored as an index of a specific effect at the cellular level and protein synthesis, RNA synthesis and cell viability can be monitored as indices of non-specific effects at the cellular level. In an intact subject, melatonin production can be monitored by measurement of the major melatonin metabolite, 6-sulfatoxymelatonin and nonspecific effects can be monitored in the subject by measuring such parameters as water intake, food intake, weight gain, locomotor activity and body temperature. More sophisticated tests can include various psychological indices, such as problem solving, memory, and aggressiveness.

The substrate derivatives of this invention can enter a cell by diffusion through the cell membrane. A particular substrate that is not readily diffusible through the cell membrane can be altered according to well known methods to increase solubility of the substrate.

In certain cases in which transport mechanisms exist for cellular uptake of specific substances, including serotonin and amino acids, the substrate derivatives of this invention can be altered according to known procedures to facilitate transportation of the substrates into a cell through such mechanisms.

It is further contemplated that the substrate derivative of this invention can be attached to a ligand. For example, a ligand can be attached to the substrate derivative in cases where it is desirable to introduce a large peptide, such as a substrate derivative of protein or histone acetyltransferases, into a cell. In this situation, a peptide is attached to a ligand that is internalized upon binding its receptor, resulting in the delivery of the peptide to the interior of the cell as part of the ligand-receptor complex.

In addition, the present invention provides for the formation of a bisubstrate inhibitor in a specific cell type by attaching the substrate derivative of this invention to the ligand of a receptor present on a specific target cell. For example, a cancer cell might be targeted for bisubstrate inhibitor formation by attaching the substrate derivative to a ligand which binds a receptor which is abundantly expressed on the surface of a specific cancer cell.

The ligands of this invention can be attached distal to the alkylating group of the substrate derivative by formation of covalent bonds using methods well known in the art.

The substrate derivatives employed in the methods of this invention can be administered to a cell either *in vivo* or *ex vivo*. Thus the substrate derivatives of the present invention can be in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects in a subject or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

If *ex vivo* methods are employed to administer a substrate derivative of this invention, cells or tissues can be removed and maintained outside the body according to standard protocols well known in the art. The substrate derivative of this invention can be introduced into the cells according to mechanisms well known in the art (e.g., diffusion, receptor mediated endocytosis), as described herein and in the available literature. The cells can then be infused (e.g., in a pharmaceutically acceptable carrier) or transplanted back into the subject per standard methods for the cell or tissue type. Standard methods are known for transplantation or infusion of various cells into a subject.

Preparations are provided for *in vivo* administration; in that case the substrate derivatives can be administered to a subject orally, parenterally (e.g., intravenously), by intramuscular injection, by intraperitoneal injection, transdermally, extracorporeally, intranasally, topically or the like. Delivery can also be directly to any area of the respiratory system (e.g., lungs) via intubation. The exact amount of the substrate derivative required will vary from subject to subject, depending on the species, age, weight and general condition of the subject, the severity of the disorder being treated, the particular substrate derivative used, its mode of administration and the like. Thus, it is not possible to specify an exact amount for every substrate derivative. However, an appropriate amount can be determined by one of ordinary skill in the art using only routine experimentation given the teachings herein and what is available in the art (21).

Parenteral administration of the substrate derivative of the present invention, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795.

In the methods of the present invention which describe the use of alkylating derivatives to prepare medicaments for the treatment of a disorder, the efficacy of the treatment can be monitored according to clinical protocols well known in the art for monitoring the treatment of the particular disorder.

For example, a human subject (patient) diagnosed with a depression is treated by oral administration of an alkylating derivative of an acetyl acceptor substrate of serotonin N-acetyltransferase (AANAT) as defined in claim 19, (e.g., bromoacetyl tryptamine or chloroacetyl tryptamine) in a dosage range from about 1 to about 10 mg per kg of body weight, 1 to 4 times a day. The patient is monitored for general physical signs to evaluate nonspecific effects of treatment and by analysis of blood chemistry to identify changes in salt balance and liver function. Efficacy of the treatment is evaluated using standard indices of depression well known in the art of psychiatry.

A non-human subject is administered a substrate derivative of this invention orally and/or by subcutaneous injection of the substrate derivative in solution or as a suspension, in a dosage range from about 1 to about 10 mg per kg body weight. The subject is monitored by evaluation of activity cycles, food intake, water intake, general behavior, posture and other such parameters as are well known in the art for evaluation of non-human subjects.

As described herein, the source of the acetyltranferase in a cell of this invention can be an exogenous nucleic acid (RNA and/or DNA) encoding the acetyltransferase. Thus, the nucleic acid of the present invention can be in a pharmaceutically acceptable carrier and can be delivered to cells *in vivo* and/or *ex vivo* by a variety of mechanisms well known in the art (e.g., uptake of naked DNA, viral infection, liposome fusion, intramuscular injection of DNA via a gene gun, endocytosis, etc.).

If *ex vivo* methods are employed to administer a nucleic acid encoding an acetyltransferase, cells or tissues can be removed from a subject and maintained outside the subject's body according to standard protocols well known in the art. The nucleic acid of this invention can be introduced into the cells via any nucleic acid transfer mechanism, such as, for example, virus-mediated nucleic acid delivery, calcium phosphate mediated nucleic acid delivery, electroporation, microinjection or proteoliposomes. The transduced cells can then be infused (e.g., in a pharmaceutically acceptable carrier) or transplanted back into the subject per standard methods for the cell or tissue type. Standard methods are well known for transplantation or infusion of various cells into a subject.

The cell to which the nucleic acid can be administered can be any cell which can take up and express exogenous nucleic acid encoding an acetyltransferase and can produce an acetyltransferase which is functional within the cell.

It is contemplated that the methods described above can include the administration and uptake of exogenous nucleic acid into the cells of a subject *in vivo* (i.e., via transduction or transfection). Such nucleic acid can be in the form of a naked nucleic acid or the nucleic acid can be in a vector for delivering the nucleic acid to the cells for expression of the nucleic acid encoding the acetyltransferase inside the cell. As one example, the vector can be a commercially available preparation, such as an adenovirus vector (Quantum Biotechnologies, Inc. (Laval, Quebec, Canada). Delivery of the nucleic acid or vector to cells can be via a variety of mechanisms, such as, via a liposome, using commercially available liposome preparations (e.g., LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI)), as well as other liposomes developed according to procedures standard in the art. In addition, the nucleic acid or vector of this invention can be delivered *in vivo* by electroporation, the technology for which is available from Genetronics, Inc. (San Diego, CA) as well as by means of a SONOPORATION machine (ImaRx Pharmaceutical Corp., Tucson, AZ).

The vector delivery of this invention can be via a viral system, such as a retroviral vector system which can package a recombinant retroviral genome (14,15). The recombinant retrovirus can then be used to infect and thereby deliver to the infected cells the nucleic acid encoding the acetyltransferase. The exact method of introducing the nucleic acid into mammalian cells is, of course, not limited to the use of retroviral vectors. Other techniques are widely available for this procedure including, but not limited to, the use of adenoviral vectors (16), adeno-associated viral (AAV) vectors (17), lentiviral vectors (18) and/or pseudotyped retroviral vectors (19). Physical transduction techniques can also be used, such as liposome delivery and receptor-mediated and other endocytosis mechanisms (20). This invention can be used in conjunction with any of these or other commonly used nucleic acid delivery methods.

The nucleic acid or vector of this invention can be administered parenterally (e.g., intravenously), by intramuscular injection, by intraperitoneal injection, transdermally, extracorporeally, intranasally, topically or the like. Delivery can also be directly to any area of the respiratory system (e.g., lungs) via intubation. The exact amount of the nucleic acid or vector required will vary from subject to subject, depending on the species, age, weight and general condition of the subject, the severity of the disorder being treated, the particular nucleic acid or vector used, its mode of administration and the like. Thus, it is not possible to specify an exact amount for every nucleic acid or vector. However, an appropriate amount can be determined by one of ordinary skill in the art using only routine experimentation given the teachings herein (21).

As one example, if the nucleic acid of this invention is delivered to the cells of a subject in an adenovirus vector, the dosage for administration of adenovirus to humans can range from about 10⁷ to 10⁹ plaque forming unit (pfu) per injection, but can be as high as 10¹² pfu per injection (22,23).

Parenteral administration of the nucleic acid or vector of the present invention, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795.

In a further embodiment, the present invention provides a cell comprising a bisubstrate inhibitor, wherein the bisubstrate inhibitor comprises an alkylating derivative of an acetyl acceptor substrate for an acetyltransferase present in the cell and CoA. As described herein, the cell can produce the acetyltransferase naturally or as the result of expression of an exogenous nucleic acid encoding the acetyltransferase.

The cell of this invention can comprise an alkylating derivative of the acetyl acceptor substrate which is a N-bromoacetylated acetyl acceptor substrate, a N-chloroacetylated acetyl acceptor substrate and/or a N-fluoroacetylated acetyl acceptor substrate. Furthermore, the cell of this invention can be *in vivo* or *ex vivo* and can thus be present in a pharmaceutically acceptable carrier.

For example, in a cell of this invention wherein the acetyltransferase is arylalkylamine N-acetyltransferase (AANAT), the alkylating derivative of the acetyl acceptor substrate of AANAT can be, but is not limited to, N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and/or N-fluoroacetyltyramine, as well as any other alkylating derivative of an acetyl acceptor substrate for AANAT that is now known or later identified. Furthermore, such a cell, wherein the acetyltransferase is AANAT, can be a pineal gland cell, a retinal cell (e.g., a photoreceptor cell and/or ganglion cell), or any other cell now known or later identified to naturally produce AANAT or to be capable of expressing an exogenous nucleic acid encoding AANAT.

It is also contemplated that the alkylating derivatives of the acetyl accepting substrates of acetyltransferases of this invention be used to modulate the acetylation of certain drugs which are inactivated by acetylation, thereby prolonging the effectiveness of the drug and/or minimizing adverse reactions which result from acetylation of certain drugs.

In particular, it is well known that certain drugs are inactivated by acetylation which occurs during the normal course of metabolism of the drug in a subject. This inactivation by acetylation can be blocked by a substrate derivative of this invention which forms a bisubstrate inhibitor of the inactivating acetyltransferase, thereby prolonging the effectiveness of the drug.

Furthermore, it is well documented that a variety of adverse drug reactions are due to acetylation of certain drugs in the cells of a subject. For example, Table 4 shows a list of drugs which are known to act as substrates for the liver N-acetyltransferase, arylamine N-acetyltransferase (E.C. 2.3.1.5) and the table matches these drugs with adverse reactions which have been reported to result from acetylation of these drugs (i.e., activation to a form that produces adverse effects).

The level of this enzyme in people is genetically determined, resulting in the classification of individuals as high acetylators or acetylators. Accordingly, the rate at which these drugs are acetylated when administered will vary significantly. Patients can be phenotyped prior to administration of the drug for identification as either a high or low level acetylator. (The heading in Table 4: Phenotyping Assay identifies those drugs which have been used to phenotype individuals). The resulting dosage of drug administered to the patient is a balance between reduced side effects and optimal drug concentration.

Co-administration of these drugs and an alkylating derivative of a drug that would generate a bisubstrate inhibitor of arylamine N-acetyltransferase 2.3.1.5 (e.g., isoniazid and N-bronioacetylisoniazid; hydralizine and N-bromoacetylhydralizine; sulfamethazine and N-bromoacetylsulfamethazine; phenetidine and N-bromoacetylphenetidine) would allow higher concentrations of the drug to be administered with reduced or no side effects.

Thus, the present invention provides an ex-vivo method and an agent for prolonging the effectiveness of a drug in a subject, comprising co-administering to the subject an effective.amount of the drug and an alkylating derivative of an acetyl acceptor substrate which is specific for an acetyltransferase which acetylates (and thereby inactivates) the drug.

Additionally, the present invention provides an ex-vivo method and an agent for reducing or preventing an adverse reaction to a drug which is caused by acetylation of the drug in a subject, comprising co-administering to the subject an effective amount of the drug and an effective amount of an alkylating derivative of the drug.

The present invention is more particularly described in the following examples which are intended as illustrative only.

### EXAMPLES

***Reagents.** N*-Bromoacetyltryptamine and CoA-*S-N*-acetyltryptamine were synthesized following published methods (4) and provided by Research Biochemicals, Co. through the National Institute of Mental Health's Drug Synthesis Program. Pineal glands were obtained from male Sprague-Dawley rats (150 gm, University of Alberta Animal unit). Other.chemicals and supplies were obtained from commercial sources, except for recombinant AANAT, which was prepared as described below.

***Preparation of AANAT.*** Preparation ofbacterial colonies containing recombinant AANAT DNA was done essentially as described (5). After centrifugation, cells from 4 liters of bacterial cultures were resuspended in 200 ml lysis buffer 1 (2 x phosphate-buffered saline [PBS], 10 mM dithiothreitol [DTT], 1 mM EDTA) and stored at -80°C. After thawing, cells were lysed by sonication at 4°C followed by centrifugation at 100,000 x g for 45 min. Twenty milliliters of glutathione SEPHAROSE 4B (Pharmacia), previously equilibrated in 1 x PBS, was added to the supernatant, and the slurry was mixed at 4°C for 1hr. The resin was then poured into a 100 ml column (BioRad) and washed with 10 column volumes of buffer 2 (1 x PBS, 0.5M NaCl, 10 mM DTT, 1 mM EDTA) followed by 10 column volumes of buffer 3 (20 mM Tris [pH 7.5], 0.5 M NaCl, 10 mM DTT, 1 mM EDTA, 10% glycerol). The protein was eluted using 10 mM glutathione in buffer 3 (pH adjusted to 8). Fractions containing the fusion protein (evaluated by absorption at 280 nm) were combined and dialyzed against buffer 3.

The dialyzed preparation was incubated with thrombin (1 unit/ml., Boehringer Mannheim) at 4°C for 12 hours. The digested sample was mixed with a suspension of glutathione-SEPHAROSE and benzamidine-SEPHAROSE and incubated for 2 hours with gentle mixing. The gel/enzyme mixture was poured into an empty column; the gel was retained and the flow-through fraction, which contained enzyme activity, was collected and concentrated. The concentrated sample was further purified by fractionation on a size exclusion column (TSK 3000, Toyasoda) equilibrated with buffer 3. The fractions containing the enzyme activity were pooled, concentrated and stored in 0.05 to 0.5 ml aliquots at - 80°C. The specific activity of this preparation was 1.5 mmoles/h/mg protein.

***AANAT assay:*** The specific activity of the AANAT preparation was determined from measurement of the activity of the enzyme and amount of protein in a preparation. Activity was assayed by incubating the enzyme with ³H-acetyl CoA (0.5 mM, 4µCi/µmole) and tryptamine hydrochloride (1 mM), bovine serum albumin (0.5 mg/ml) and the enzyme in total volume of 100 µl of sodium phosphate (0.1M, pH 6.8) (6,7). The incubation was terminated by extracting the product ³H-acetyltryptamine with chloroform (1 ml). The chloroform phase was washed sequentially with 200 µl of the sodium phosphate buffer and twice with 200 µl of NaOH (IN). The radioactivity in 400 µl of the chloroform phase was determined following evaporation of chloroform under vacuum. Protein was measured by optical density at 280 nm and by dye binding using the Brandford procedure.

***Mass spectral analysis:*** Matrix assisted laser desorption/ionization time-of-flight mass spectrometry (MALDI-TOF MS) analysis was done by mixing 1 µl of the aqueous phase from the chloroform extraction with an equal volume of α-cyano-4-hydroxycinnamic acid (saturated in 50% CH₃CN, 0.1% TFA). The mixture was applied to the MALDI-TOP MS stage. Mass spectra were acquired in negative ion mode on a PerSeptive Biosystems Voyager DE mass spectrometer (8,9). Each spectrum represents the summed average of 100 laser shots.

*Pineal cell preparation and treatment:* Pinealocytes were prepared from rat pineal glands by trypsinization as previously described (10). The cells were suspended in Dulbecco's modified Eagle medium (DMEM) containing 10% fetal calf serum and maintained (37°C) for 18 h in a gas mixture of 95% air and 5% CO₂. During this 18 hour period, some cells were treated, as indicated in Table 2, experiment 2, in aliquots of 50,000 cells/300 µl and washed prior to addition of fresh medium and further treatment. In some cases, as in Table 3, experiment 1, cells were not aliquoted until after 18 hours of control incubation and in these cases, aliquots of cells (50,000 cells/300 µl) were prepared and treated with drugs. Drugs were prepared in 100x concentrated solutions in water or dimethyl sulfoxide. The duration of the drug treatment was 5 h.

At the end of treatment period, cells were collected by centrifugation (2 min, 10,000g) and the medium was removed and stored. Cell pellets were frozen immediately in dry ice and stored at -80° C until determination of AANAT activity. The supernatant collected was used for determination of melatonin.

***Melatonin analysis.*** Melatonin in the medium was determined by a radioimmunoassay as described previously (11,12). Briefly, melatonin was extracted from 300 µl of medium by vortexing with 1 ml of methylene chloride. After centrifugation, 700 µl of the organic phase was collected and evaporated to dryness. The residue was reconstituted in 500 µl of assay buffer (0.01M phosphate buffer, pH 7.5, containing 0.1% gelatin).

An ultraspecific melatonin antiserum from CIDtech Research Inc. (Mississauga, Ontario Canada) was used for the melatonin radioimmunoassay. Aliquots of extracted melatonin samples were diluted to 500 µl with assay buffer. 100 µl of melatonin antiserum and 50 µl of [³H]melatonin (~2000cpm, at 45.5Ci/mmol) were then added. After mixing, the tubes were incubated at 4°C overnight. To separate the bound from the free [³H] melatonin, 650 µl of saturated ammonium sulphate were added and the samples were incubated at 4°C for 1 hr. The bound [³H]melatonin was collected in a protein pellet by centrifugation (4,000g x 30 min) and re-dissolved in 550 µl of deionized water. 500 µl was then used for scintillation counting. Both inter- and intra-assay variability were less than 10 %.

***Incubations to study formation of CoA-S-N-acetyltryptamine.*** -Bromoacetyltryptamine (20 µM) was incubated (37°C, 30 min) with coenzyme A (20 µM), bovine serum albumin (0.5 mg/ml) and the enzyme in a total volume of 100 µl of Tris HCl (50 mM, pH 7.4) and the incubation was terminated by adding chloroform (1 ml) and vortexing. The aqueous phase was then removed. Samples of the aqueous phase from each chloroform extraction were analyzed by mass spectroscopy and for inhibitory activity in an AANAT assay.

***AANAT catalyzes the formation of CoA-S-N-acetyltryptamine from N-bromoacetyltryptamine and CoA.*** MALDI-TOF MS analysis revealed that a sample containing ~10 picomoles of authentic synthetic CoA-*S*-*N*-acetyltryptamine generates a spectral pattern characterized by a strong peak at *967 m*/*z*. A compound generating a peak with the same *m*/*z* was also present in samples of AANAT incubated with CoA and *N*-bromoacetyltryptamine. This compound was not present in samples that did not contain AANAT, nor in those that did not contain CoA, nor in those that did not contain *N*-bromoacetyltryptamine, nor in samples that contained boiled AANAT, nor in those that were not incubated. An inactive form of AANAT which carries a Y168F mutation (13) was also shown not to catalyze the formation of CoA-*S*-*N*-acetyltryptamine in this type of experiment. The results in Figure 2 provide physical evidence that active AANAT catalyzes the formation of the potent AANAT inhibitor CoA-*S*-*N*-acetyltryptamine from CoA and *N*-bromoacetyltryptamine.

This conclusion was supported by the biochemical evidence from experiments in which the aqueous extracts analyzed above were incubated with a fresh preparation of AANAT in an AANAT assay. Inhibitory activity of the extract was determined. It was found that there was a direct correlation between the levels of CoA-*S*-*N*-acetyltryptamine in the samples from Figure 2 and the ability to inhibit AANAT activity (Table 2).

The physical and biochemical evidence above indicate that AANAT catalyzes the formation of the potent AANAT inhibitor CoA-*S*-*N*-acetyltryptamine from CoA and *N*-bromoacetyltryptamine.

***N-Bromoacetyltryptamine treatment of rat pinealocytes inhibits melatonin production***. Experiments were conducted to determine if N-bromoacetyltryptamine could inhibit production of melatonin. This was done using rat pinealocytes in which melatonin production was elevated by treatment with norepinephrine, which acts by elevating the activity of AANAT.

It was found that a 5 hour treatment with 0.1, 0.5 or 1 µM concentration of *N*-bromoacetyltryptamine markedly reduced the production of melatonin (Table 3). The effect of an 18 hour treatment with 0.5 µM *N*-bromoacetyltryptamine was examined. Following the 18 hour treatment, the medium was changed and fresh medium was added. Following this treatment, cells responded to norepinephrine treatment with an increase in melatonin production and AANAT activity. This indicates that an 18 hour treatment with 0.5 µM *N*-bromoacetyltryptamine is not cytotoxic and that inhibition of melatonin production seen in norepinephrine-treated cells reflects the effect of CoA-*S-N*-acetyltryptamine on melatonin production and does not reflect cell death.

### REFERENCES

1. Klein, D.C., Coon, S.L., Roseboom, P.H., Weller, J.L.,Bernard, M., Gastel, J.A., Zatz, M., Iubone, M., Rodriquez, I.R., Begάy, V., Falcon, J., Cahill, G., Cassone, V.M. and Baler, R. (1997) The melatonin rhythm generating enzyme: Molecular regulation of serotonin *N*-acetyltransferase in the pineal gland. *Recent Progress in Hormone Research* 52: 307-358.
2. Klein, D.C., Roseboom, P.H. and Coon, S.L. (1996) New light is shining on the melatoninrhythm enzyme: The first post cloning view. *Trends in Endocrinology and Metabolism* 7:106-112.
3. Arendt, J. (1995) *Melatonin and the mammalian pineal gland.* Chapman and Hall, London.
4. Khalil, E.M. and Cole, P.A. (1998). A potent inhibitor of melatonin rhythm enzyme. *J.Am. Chem. Soc.* 120: 6195-6196.
5. Dunghills, J., Gastel, J., Klein, D.C. and Cole, P.A. (1998). Kinetic analysis of the catalytic mechanism of serotonin N-acetyltransferase (E.C. 2.3.1.87). *J. Biol. Chem.* 273:3045-3050.
6. Dogwatch, T. and Axelrodi, J. (1972). Sensitive assay for serotonin N-acetyltransferase activity in rat pineal. *Anal. Became* 50:174-179.
7. Namboodiri, M.A.A., Dubbels, R. and Klein, D.C. (1987) Arylalkylamine N-acetyltransferase from mammalian pineal gland. *Meth. Enzymol*. 142:538-590.
8. Hillenkamp. F., Kara, M., Beavis, R.C. and Chait, B.T. (1991) Matrix-assisted laser desorption/ionization mass spectrometry ofbiomolecules. *Anal. Chem. 63:* 1193A-1202A.
9. Hillenkamp, F. and Karas, M., (1990) Mass spectrometry of peptides and proteins by matrix-assisted ultraviolet laser desorption/ionization. *Methods Enzymol*. 193:280-295.
10. Buda M. and Klein, D.C. (1978) A suspension culture of pinealocytes: Regulation of N-acetyltransferase activity. *Endocrinology* 103: 1483-1493.
11. Ho, A.K., Grota, L.J. and Brown, G.B. (1984) Relationship between pineal N-acetyltransferase activity, pineal melatonin and serum melatonin in rats under different lighting conditions. *Neuroendocrinology* 39, 465-470.
12. Chik, L.C. and Ho, A.K. (1992) Ethanol reduces norepinephrine-stimulated melatonin synthesis in rat pinealocytes. *J. Neurochem.* 59 1280-1286.
13. Hickman, A. B., Namboodiri, M.A.A., Klein, D.C. and Dyda, F. (1999) The structural basis of ordered substrate binding by serotonin N-acetyltransferase: Complex at 1.8Å resolution with a bisubstrate inhibitor. *Cell* 97:361-369.
14. Pastan et al. A retrovirus carrying an MDR1 cDNA confers multidrug resistance and polarized expression of P-glycoprotein in MDCK cells. *Proc. Nat. Acad. Sci.* 85:4486 (1988).
15. Miller et al. Redesign of retrovirus packaging cell lines to avoid recombination leading to helper virus production. *Mol. Cell Biol*. 6:2895 (1986).
16. Mitani et al. Transduction of human bone marrow by adenoviral vector. *Human Gene Therapy* 5:941-948 (1994).
17. Goodman et al. Recombinant adeno-associated virus-mediated gene transfer into hematopoietic progenitor cells. *Blood* 84:1492-1500 (1994)
18. Naidini et al. *In vivo* gene delivery and stable transduction of nondividing cells by a lentiviral vector. *Science* 272:263-267 (1996)
19. Agrawal et al. Cell-cycle kinetics and VSV-G pseudotyped retrovirus mediated gene transfer in blood-derived CD34⁺ cells. *Exp. Hematol*. 24:738-747 (1996).
20. Schwarzenberger et al. Targeted gene transfer to human hematopoietic progenitor cell lines through the *c-kit* receptor. *Blood* 87:472-478 (1996).
21. Martin, E.W. (ed.) *Remington's Pharmaceutical Sciences,* latest edition. Mack Publishing Co., Easton, PA.
22. Crystal, R.G. 1997. Phase I study of direct administration of a replication deficient adenovirus vector containing *E. coli* cytosine deaminase gene to metastatic colon carcinoma of the liver in association with the oral administration of the pro-drug 5-fluorocytosine. *Human Gene Therapy* 8:985-1001.
23. Alvarez, R.D. and D.T. Curiel. 1997. A phase I study of recombinant adenovirus vector-mediated delivery of an anti-erbB-2 single chain (sFv) antibody gene from previously treated ovarian and extraovarian cancer patients. *Hum. Gene Ther*. **8**:229-242.

**TABLE 1.**

| Enzyme Code # | Enzyme | Inhibitor |
|---|---|---|
| 2.3.1.1 | Amino-acid N-acetyltransferase. | N-bromoacetyl amino acids |
| 2.3.1.2 | Imidazole N-acetyltransferase. | N-bromoacetylimidazole |
| 2.3.1.3 | Glucosamine N-acetyltransferase. | N-bromoacetylglucosamine |
| 2.3.1.4 | Glucosamine-phosphate N-acetyltransferase. | N-bromoacetylglucosaminephosphate |
| 2.3.1.5 | Arylamine N-acetyltransferase. | N-bromoacetylphenetidine N-bromoacetylisoniazid |
| 2.3.1.6 | Choline O-acetyltransferase. | O-bromoacetylcholine |
| 2.3.1.7 | Carnitine O-acetyltransferase. | O-bromoacetylcarnitine. |
| 2.3.1.11 | Thioethanolamine S-acetyltransferase. | S-bromoacetylthioethanolamine |
| 2.3.1.12 | Dihydrolipoamide S-acetyltransferase. | S-bromoacetyl-Dihydrolipoamide |
| 2.3.1.13 | Glycine N-acyltransferase. | N-bromoacetylglycine |
| 2.3.1.14 | Glutamine N-phenylacetyltransferase. | N-bromophenylacetylglutamine |
| 2.3.1.15 | Glycerol-3-phosphate O-acyltransferase. | O-bromoacetylglycerol-3-phosphate |
| 2.3.1.17 | Aspartate N-acetyltransferase. | N-bromoacetylaspartate |
| 2.3.1.18 | Galactoside O-acetyltransferase. | O-bromoacetylgalactoside |
| 2.3.1.21 | Carnitine O-palmitoyltransferase. | O-bromopalmitoylcarnitine |
| 2.3.1.24 | Sphingosine N-acyltransferase. | N-bromoacetylsphingosine |
| 2.3.1.27 | Cortisol O-acetyltransferase. | O -bromoacetylcortisol |
| 2.3.1.28 | Chloramphenicol O-acetyltransferase. | O -bromoacetylchloramphenicol |
| 2.3.1.29 | Glycine C-acetyltransferase. | C-bromoacetylglycine |
| 2.3.1.30 | Serine O-acetyltransferase. | O-bromoacetylserine |
| 2.3.1.31 | Homoserine O-acetyltransferase. | O-bromoacetylhomoserine |
| 2.3.1.32 | Lysine N-acetyltransferase. | N-bromoacetyllysine |
| 2.3.1.33 | Histidine N-acetyltransferase. | N-bromoacetylhistidine |
| 2.3.1.34 | D-tryptophan N-acetyltransferase. | N-bromoacetyl D-tryptophan |
| 2.3.1.35 | Glutamate N-acetyltransferase. | N-bromoacetylglutamate |
| 2.3.1.36 | D-amino-acid N-acetyltransferase. | N-bromoacetyl D-amino acids |
| 2.3.1.46 | Homoserine O-succinyltransferase. | O-bromosuccinylhomoserine |
| 2.3.1.50 | Serine C-palmitoyltransferase. | C-bromopalmitoylserine |
| 2.3.1.51 | 1-acylglycerol-3-phosphate O-acyltransferase. | O-bromoacetyl 1-acylglycerol-3-phosphate |
| 2.3.1.52 | 2-acylglycerol-3-phosphate O-acyltransferase. | O-bromoacetyl 2-acylglycerol-3 phosphate |
| 2.3.1.53 | Phenylalanine N-acetyltransferase. | N-bromoacetylphenylalanine |
| 2.3.1.54 | Formate C-acetyltransferase. | C-bromoacetylformate |
| 2.3.1.56 | Aromatic-hydroxylamine O-acetyltransferase. | O-bromoacetyl aromatic hydroxylamines |
| 2.3.1.57 | Diamine N-acetyltransferase. | N-bromoacetyldiamines including spermidine and spermine |
| 2.3.1.59 | Gentamicin 2'-N-acetyltransferase. | 2'-N-bromoacetyl gentamicin |
| 2.3.1.60 | Gentamicin 3'-N-acetyltransferase. | 3'-N-bromoacetyl gentamicin |
| 2.3.1.61 | Dihydrolipoamide S-succinyltransferase. | S-bromosuccinyl dihydrolipoamide |
| 2.3.1.64 | Agmatine N4-coumaroyltransferase. | N4-bromocoumaroyl-agmatine |
| 2.3.1.65 | Glycine N-choloyltransferase. | N-bromocholoylglycine |
| 2.3.1.66 | Leucine N-acetyltransferase. | N-bromoacetylleucine |
| 2.3.1.68 | Glutamine N-acyltransferase. | N-bromoacetylglutamine |
| 2.3.1.71 | Glycine N-benzoyltransferase. | N-bromobenzoylglycine |
| 2.3.1.80 | Cysteine-S-conjugate N-acetyltransferase. | N-bromoacetyl cysteine-S-conjugate |
| 2.3.1.81 | Aminoglycoside N3'-acetyltransferase. | N-bromoacetyl-N3'-aminoglycoside |
| 2.3.1.82 | Kanamycin 6'-N-acetyltransferase. | 6'-N-bromoacetylkanamycin |
| 2.3.1.87 | Aralkylamine N-acetyltransferase. | N-bromoacetyltryptamine N-bromoacetylphenyethylamine |
| 2.3.1.88 | Peptide alpha-N-acetyltransferase. | Alpha-N-bromoacetyl peptide |
| 2.3.1.102 | N6-hydroxylysine O-acetyltransferase. | O-bromoacetyl N6-hydroxylysine |
| 2.3.1.104 | 1-alkenylglycerophosphocholine O-acyltransferase | O-bromoacetyl 1-alkenyl-Glycerophosphocholine |
| 2.3.1.109 | Arginine N-succinyltransferase. | N-bromosuccinylarginine |
| 2.3.1.110 | Tyramine N-feruloyltransferase. | N-bromoferuloyltyramine |
| 2.3.1.112 | D-tryptophan N-malonyltransferase. | N-bromomalonyl-D-tryptophan |
| 2.3.1.113 | Anthranilate N-malonyltransferase. | N-bromomalonylanthranilate |
| 2.3.1.114 | 3,4-dichloroaniline N-malonyltransferase. | N-bromomalonyl 3,4-dichloroaniline |
| 2.3.1.118 | N-hydroxyarylamine O-acetyltransferase. | O-bromoacetyl N-hydroxyarylamines |
| 2.3.1.127 | Ornithine N-benzoyltransferase. | N-bromobenzoylornithine |
| 2.3.1.133 | Shikimate O-hydroxycinnamoyltransferase. | O-bromohydroxycinnamoylshikimate |
| 2.3.1.135 | Phosphatidylcholine--retinol O-acyltransferase. | O-bromoacetylphosphatidylcholine -retinol |
| 2.3.1.137 | Carnitine O-octanoyltransferase. | O-bromo-octanoylcamitine |
| 2.3.1.139 | Ecdysone O-acyltransferase. | O-bromoacetylecdysone |
| 2.3.1.144 | Anthranilate N-benzoyltransferase. | N-bromobenzoylanthranilate |
| 2.3.1.145 | Piperidine N-piperoyltransferase. | N-bromopiperoyl-piperidine |
| 2.3.1.150 | Salutaridinol 7-O-acetyltransferase. | 7-O-bromoacetylsalutaridinol |

**TABLE 2.**

| | |
|---|---|
| AANAT-catalyzed synthesis of its inhibitor from CoA and *N*-bromoacetyltryptamine. To determine if the chloroform extracted aqueous phases of the reactions from Figure 2 contained compounds that inhibited AANAT activity, a 20 µl sample was added to a 100 µl AANAT assay, as described herein. | |

| **Pre-AANAT assay reaction conditions** | **AANAT Activity (% of control)** |
|---|---|
| Complete | 38% |
| Minus *N*-bromoacetyltryptamine | 98% |
| Minus CoA | 107% |
| Zero time | 110% |
| Boiled enzyme | 104% |

**TABLE 3.**

| | | | |
|---|---|---|---|
| Effect of *N*-bromoacetyltryptamine on melatonin production by norepinephrine-treated pinealocytes and effects on stimulation of AANAT activity. Cells were prepared and treated as described herein. Experiment 1 shows that 0.1 or 1.0 µM *N*-bromoacetyltryptamine treatment inhibits melatonin production during a 5 hour test period. Experiment 2 shows that, after an 18 hour treatment period with 0.5 µM *N*-bromoacetyltryptamine(BAT) and subsequent wash out to remove the drug, pinealocytes are still able to respond to norepinephrine (NE) with an increase in AANAT activity, indicating that they have not been killed by prior treatment. | | | |

| Experiment 1. | | | |
|---|---|---|---|
| **Treatment of pinealocytes in culture (18-24 hours)** | | | **Melatonin production (pmol/100,000 cells, 18-24 hours)** |
| Control | | | Not detectable |
| Norepinephrine (10 µM) | | | 12.43 ± 2.00 |
| *N*-Bromoacetyltryptamine (1 µM) | | | 0.65 ± 0.05 |
| Norepinephrine (10 µM) +*N*-Bromoacetyltryptamine (1 µM) | | | 0.95 ± 0.25 |
| Norepinephrine (10 µM) +*N*-Bromoacetyltryptamine (0.1 µM) | | | 4.55 ± 0.04 |

| Experiment 2. | | | |
|---|---|---|---|
| **Treatment I (0-18 hr)** | **Treatment II (18-23 hr)** | **Melatonin (18-23 hr) (pmole/10**^{**5**} **cells)** | **AANAT (23 hr) (µmol/h/105 cells)** |
| DMSO | Control | 1.12 ± 0.31 | ND |
| DMSO | NE 10 µM | 10.44 ± 1.07 | 0.89 ± 0.10 |
| DMSO | NE 10 µM + BAT (0.5µM) | 1.88 ± 0.82 | 0.48 ± 0.05 |
| BAT (0.5 µM) | Control | 1.78 ± 0.14 | ND |
| BAT (0.5 µM) | NE (10 µM) | 12.22 ± 2.90 | 0.86 ± 0.06 |

**TABLE 4.**

| **MEDICATIONS ACTIVATED BY N-ACETYLTRANSFERASE SOME EXAMPLES** | | |
|---|---|---|
| Medication | Adverse Drug Reaction Reported | Phenotyping Assay |
| Isoniazid | Peripheral neuropathy, liver | + |
| | disease | |
| Hydralazine | Lupus, uncontrolled hypertension | + |
| Procainamide | Lupus, uncontrolled arrhythmias | |
| Dapsone | Hematological | + |
| Caffeine | | + |
| Clonazepam | | |
| Aminoglutethamide | Adrenal insufficiency | |
| Sulfamethazine | Hematological/Gastrointestinal | + |
| Sulfapyridine | | |
| Amrinone | | |

## Claims

1. An ex-vivo method of producing a bisubstrate inhibitor in a cell, comprising introducing into the cell an alkylating derivative of an acetyl acceptor substrate for an acetyltransferase present in the cell.

2. The method of claim 1, wherein the acetyltransferase is produced by the cell.

3. The method of claim 1, wherein the acetyltransferase is produced in a cell from an exogenous nucleic acid encoding the acetyltransferase.

4. The method of claim 1, wherein the alkylating derivative of the acetyl acceptor substrate is selected from the group consisting of a N-bromoacetylated acetyl acceptor substrate, a N-chloroacetylated acetyl acceptor substrate and a N-fluoroacetylated acetyl acceptor substrate.

5. The method of claim 1, wherein the acetyltransferase is arylalkylamine N-acetyltransferase (AANAT) and the alkylating derivative of the acetyl acceptor substrate is selected from the group consisting of N-bronioacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and N-fluoroacetyltyramine.

6. An ex-vivo method of inhibiting the activity of an acetyltransferase in a cell, comprising introducing into the cell an alkylating derivative of an acetyl acceptor substrate for an acetyltransferase present in the cell under conditions whereby a bisubstrate inhibitor will be produced, thereby inhibiting the activity of the acetyltransferase in the cell.

7. The method of claim 6, wherein the acetyltransferase is produced by the cell.

8. The method of claim 6, wherein the acetyltransferase is produced in a cell from an exogenous nucleic acid encoding the acetyltransferase.

9. The method of claim 6, wherein the alkylating derivative of the acetyl acceptor substrate is selected from the group consisting of a N-bromoacetylated acetyl acceptor substrate, a N-chloroacetylated acetyl acceptor substrate and a fluoroacetylated acetyl acceptor substrate.

10. The method of claim 6, wherein the acetyltransferase is arylalkylamine N-acetyltransferase (AANAT) and the alkylating derivative of the acetyl acceptor substrate is selected from the group consisting of N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and N-fluoroacetyltyramine.

11. An ex-vivo method of inhibiting melatonin production in a cell which produces melatonin, comprising introducing into the cell an alkylating derivative of the acetyl acceptor substrate of AANAT which is selected from the group consisting of N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and N-fluoroacetyltyramine.

12. An ex-vivo method of increasing the amount of serotonin in a cell which produces serotonin, comprising introducing into the cell an alkylating derivative of the acetyl acceptor substrate of AANAT which is selected from the group consisting of N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and N-fluoroacetyltyramine.

13. A cell comprising a bisubstrate inhibitor, wherein the bisubstrate inhibitor comprises an alkylating derivative of an acetyl acceptor substrate for an acetyltransferase present in the cell and CoA.

14. The cell of claim 13, wherein the acetyltransferase is produced by the cell.

15. The cell of claim 13, wherein the acetyltransferase is produced in the cell from an exogenous nucleic acid encoding the acetyltransferase.

16. The cell of claim 13, wherein the alkylating derivative of the acetyl acceptor substrate is selected from the group consisting of a N-bromoacetylated acetyl acceptor substrate, a N-chloroacetylated acetyl acceptor substrate and a N-fluoroacetylated acetyl acceptor substrate.

17. The cell of claim 13, wherein the acetyltransferase is arylalkylamine N-acetyltransferase (AANAT) and the alkylating derivative of the acetyl acceptor substrate is selected from the group consisting of N-bromoacetyltryptamine, N-bromoacetylserotonin, N-bromoacetylphenylethylamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoro-acetyl-methoxytryptamine and N-fluoroacetyltyramine.

18. The cell of claim 17, wherein the cell is selected from the group consisting of a pineal gland cell and a retinal cell.

19. Alkylating derivative of an acetyl acceptor substrate for an acetyltansferase in the form of a N-halogenoacetylated acetyl acceptor substrate wherein halogen is one of chloro, fluoro or bromo, for use in a medical treatment

20. Alkylating derivative of claim 19 which is selected from the group consisting of N-bromoacetyltryptamine, N-bromoacetylserotonin, N-bromoacetylphenylethytamine, N-bromo-acetyl-methoxytryptamine, N-bromoacetyltyramine, N-chloroacetyltryptamine, N-chloroacetylserotonin, N-chloroacetylphenylethylamine, N-chloro-acetyl-methoxytryptamine, N-chloroacetyltyramine, N-fluoroacetyltryptamine, N-fluoroacetylserotonin, N-fluoroacetylphenylethylamine, N-fluoroacetyl-methoxytryptamine and/or N-fluoroacetyltyramine.

21. Alkylating derivative of claim 19 for producing a bisubstrate inhibitor in a cell.

22. Alkylating derivative of claim 19 for inhibiting the activity of an acetyltransferase in a cell.

23. Derivative of one of claims 19 to 22, wherein the derivative is selected from the group defined in claim 4.

24. Derivative of one of claims 19 to 22, wherein the derivative is an alkylating derivative of an acetyl acceptor substrate for arylalkylamine N-acetyl transferase and wherein the derivative is selected from the group defined in claim 5.

25. Alkylating derivative of claim 24 for inhibiting melatonin production in a cell which produces melatonin.

26. Alkylating derivative of claim 24 for increasing the amount of serotonin in a cell which produces serotonin.

27. Alkylating derivative of claim 19 for use in a medicament for treating a disorder selected from the group consisting of depression, obsessive compulsive disorder, schizophrenia, mania, sleep/wake disorder, panic attack, migraine headache, cluster headache, insomnia, bipolar disease and attention disorder.

28. Use of an alkylating derivative of an acetyl acceptor substrate for an acetyl transferase present in the cell for preparing a medicament to produce a bisubstrate inhibitor in a cell.

29. The use of claim 28 wherein the cell is an acetyltransferase producing cell.

30. The use of claim 28 wherein the cell is producing acetyltransferase from an exogenous nucleic acid encoding the acetyltransferase.

31. The use of claim 28 wherein the alkylating derivative is selected from the group defined in claim 4.

32. The use of claim 28 wherein the acetyltransferase is arylalkylamine N-acetyltransferase (AANAT) and the alkylating derivative is selected from the group defined in claim 5.

33. The use of one of claims 28 to 32 for preparing a medicament for inhibiting the activity of an acetyltransferase in a cell.

34. The use of one of claims 28 to 32 for preparing a medicament for treating a disorder caused by a decreased amount of serotonin.

35. The use of claim 28 for preparing a medicament for inhibiting melatonin production in cell which produces melatonin.

36. Use of claim 28 for producing a medicament treating a disorder selected from the group consisting of depression, obsessive compulsive disorder, schizophrenia, mania, sleep/wake disorder, panic attack, migraine headache, cluster headache, insomnia, bipolar disease and attention disorder..

## Patentansprüche

1. Ex-vivo-Verfahren zur Herstellung eines Bisubstratinhibitors in einer Zelle, das beinhaltet, dass in die Zelle ein alkylierendes Derivat eines Acetylakzeptorsubstrats für eine Acetyltransferase, die in der Zelle vorhanden ist, eingeführt wird.

2. Verfahren nach Anspruch 1, wobei die Acetyltransferase von der Zelle erzeugt wird.

3. Verfahren nach Anspruch 1, wobei die Acetyltransferase in einer Zelle von einer exogenen Nucleinsäure, die die Acetyltransferase codiert, erzeugt wird.

4. Verfahren nach Anspruch 1, wobei das alkylierende Derivat des Acetylakzeptorsubstrats ausgewählt ist aus der Gruppe bestehend aus einem N-bromacetylierten Acetylakzeptorsubstrat, einem N-chloracetylierten Acetylakzeptorsubstrat und einem N-fluoracetylierten Acetylakzeptorsubstrat.

5. Verfahren nach Anspruch 1, wobei die Acetyltransferase Arylalkylamin-N-acetyltransferase (AANAT) ist und das alkylierende Derivat des Acetylakzeptorsubstrats ausgewählt ist aus der Gruppe bestehend aus N-Bromacetyltryptamin, N-Bromacetylserotonin, N-Bromacetylphenylethylamin, N-Bromacetylmethoxytryptamin, N-Bromacetyltyramin, N-Chloracetyltryptamin, N-Chloracetylserotonin, N-Chloracetylphenylethylamin, N-Chloracetylmethoxytryptamin, N-Chloracetyltyramin, N-Fluoracetyltryptamin, N-Fluoracetylserotonin, N-Fluoracetylphenylethylamin, N-Fluoracetylmethoxytryptamin und N-Fluoracetyltyramin.

6. Ex-vivo-Verfahren zur Hemmung der Aktivität einer Acetyltransferase in einer Zelle, das beinhaltet, dass in die Zelle ein alkylierendes Derivat eines Acetylakzeptorsubstrats für eine Acetyltransferase, die in der Zelle vorhanden ist, unter Bedingungen eingeführt wird, unter denen ein Bisubstratinhibitor erzeugt wird, wodurch die Aktivität der Acetyltransferase in der Zelle gehemmt wird.

7. Verfahren nach Anspruch 6, wobei die Acetyltransferase in der Zelle erzeugt wird.

8. Verfahren nach Anspruch 6, wobei die Acetyltransferase in einer Zelle von einer exogenen Nucleinsäure, die die Acetyltransferase codiert, erzeugt wird.

9. Verfahren nach Anspruch 6, wobei das alkylierende Derivat des Acetylakzeptorsubstrats ausgewählt ist aus der Gruppe bestehend aus einem N-bromacetylierten Acetylakzeptorsubstrat, einem N-chloracetylierten Acetylakzeptorsubstrat und einem N-fluoracetylierten Acetylakzeptorsubstrat.

10. Verfahren nach Anspruch 6, wobei die Acetyltransferase Arylalkylamin-N-acetyltransferase (AANAT) ist und das alkylierende Derivat des Acetylakzeptorsubstrats ausgewählt ist aus der Gruppe bestehend aus N-Bromacetyltryptamin, N-Bromacetylserotonin, N-Bromacetylphenylethylamin, N-Bromacetylmethoxytryptamin, N-Bromacetyltyramin, N-Chloracetyltryptamin, N-Chloracetylserotonin, N-Chloracetylphenylethylamin, N-Chloracetylmethoxytryptamin, N-Chloracetyltyramin, N-Fluoracetyltryptamin, N-Fluoracetylserotonin, N-Fluoracetylphenylethylamin, N-Fluoracetylmethoxytryptamin und N-Fluoracetyltyramin.

11. Ex-vivo-Verfahren zur Hemmung der Melatoninproduktion in einer Zelle, die Melatonin erzeugt, das beinhaltet, dass in die Zelle ein alkylierendes Derivat des Acetylakzeptorsubstrats von AANAT eingeführt wird, das ausgewählt ist aus der Gruppe bestehend aus N-Bromacetyltryptamin, N-Bromacetylserotonin, N-Bromacetylphenylethylamin, N-Bromacetylmethoxytryptamin, N-Bromacetyltyramin, N-Chloracetyltryptamin, N-Chloracetylserotonin, N-Chloracetylphenylethylamin, N-Chloracetylmethoxytryptamin, N-Chloracetyltyramin, N-Fluoracetyltryptamin, N-Fluoracetylserotonin, N-Fluoracetylphenylethylamin, N-Fluoracetylmethoxytryptamin und N-Fluoracetyltyramin.

12. Ex-vivo-Verfahren zur Erhöhung der Menge an Serotonin in einer Zelle, die Serotonin erzeugt, das beinhaltet, dass in die Zelle ein alkylierendes Derivat des Acetylakzeptorsubstrats von AANAT eingeführt wird, das ausgewählt ist aus der Gruppe bestehend aus N-Bromacetyltryptamin, N-Bromacetylserotonin, N-Bromacetylphenylethylamin, N-Bromacetylmethoxytryptamin, N-Bromacetyltyramin, N-Chloracetyltryptamin, N-Chloracetylserotonin, N-Chloracetylphenylethylamin, N-Chloracetylmethoxytryptamin, N-Chloracetyltyramin, N-Fluoracetyltryptamin, N-Fluoracetylserotonin, N-Fluoracetylphenylethylamin, N-Fluoracetylmethoxytryptamin und N-Fluoracetyltyramin.

13. Zelle enthaltend einen Bisubstratinhibitor, wobei der Bisubstratinhibitor ein alkylierendes Derivat eines Acetylakzeptorsubstrats für eine Acetyltransferase, die in der Zelle vorhanden ist, und CoA aufweist.

14. Zelle nach Anspruch 13, wobei die Acetyltransferase von der Zelle erzeugt wird.

15. Zelle nach Anspruch 13, wobei die Acetyltransferase in der Zelle von einer exogenen Nucleinsäure, die Acetyltransferase codiert, erzeugt wird.

16. Zelle nach Anspruch 13, wobei das alkylierende Derivat des Acetylakzeptorsubstrats ausgewählt ist aus der Gruppe bestehend aus einem N-bromacetylierten Acetylakzeptorsubstrat, einem N-chloracetylierten Acetylakzeptorsubstrat und einem N-fluoracetylierten Acetylakzeptorsubstrat.

17. Zelle nach Anspruch 13, wobei die Acetyltransferase Arylalkylamin-N-acetyltransferase (AANAT) ist und das alkylierende Derivat des Acetylakzeptorsubstrats ausgewählt ist aus der Gruppe bestehend aus N-Bromacetyltryptamin, N-Bromacetylserotonin, N-Bromacetylphenylethylamin, N-Bromacetylmethoxytryptamin, N-Bromacetyltyramin, N-Chloracetyltryptamin, N-Chloracetylserotonin, N-Chloracetylphenylethylamin, N-Chloracetylmethoxytryptamin, N-Chloracetyltyramin, N-Fluoracetyltryptamin, N-Fluoracetylserotonin, N-Fluoracetylphenylethylamin, N-Fluoracetylmethoxytryptamin und N-Fluoracetyltyramin.

18. Zelle nach Anspruch 17, wobei die Zelle ausgewählt ist aus der Gruppe bestehend aus einer Epiphysenzelle und einer Netzhautzelle.

19. Alkylierendes Derivat eines Acetylakzeptorsubstrats für eine Acetyltransferase in Form eines N-halogenacetylierten Acetylakzeptorsubstrats, wobei das Halogen Chlor, Fluor oder Brom ist, zur Verwendung in einer medizinischen Behandlung.

20. Alkylierendes Derivat nach Anspruch 19, das ausgewählt ist aus der Gruppe bestehend aus N-Bromacetyltryptamin, N-Bromacetylserotonin, N-Bromacetylphenylethylamin, N-Bromacetylmethoxytryptamin, N-Bromacetyltyramin, N-Chloracetyltryptamin, N-Chloracetylserotonin, N-Chloracetylphenylethylamin, N-Chloracetylmethoxytryptamin, N-Chloracetyltyramin, N-Fluoracetyltryptamin, N-Fluoracetylserotonin, N-Fluoracetylphenylethylamin, N-Fluoracetylmethoxytryptamin und N-Fluoracetyltyramin.

21. Alkylierendes Derivat nach Anspruch 19 zur Herstellung eines Bisubstratinhibitors in einer Zelle.

22. Alkylierendes Derivat nach Anspruch 19, um die Aktivität einer Acetyltransferase in einer Zelle zu hemmen.

23. Derivat nach einem der Ansprüche 19 bis 22, wobei das Derivat ausgewählt ist aus der Gruppe, die in Anspruch 4 definiert ist.

24. Derivat nach einem der Ansprüche 19 bis 22, wobei das Derivat ein alkylierendes Derivat eines Acetylakzeptorsubstrats für Arylalkylamin-N-acetyltransferase ist und wobei das Derivat ausgewählt ist aus der in Anspruch 5 definierten Gruppe.

25. Alkylierendes Derivat nach Anspruch 24, um die Melatoninproduktion in einer Zelle, die Melatonin erzeugt, zu hemmen.

26. Alkylierendes Derivat nach Anspruch 24, um die Menge an Serotonin in einer Zelle, die Serotonin erzeugt, zu erhöhen.

27. Alkylierendes Derivat nach Anspruch 19 zur Verwendung in einem Arzneimittel zur Behandlung einer Störung ausgewählt aus der Gruppe bestehend aus Depression, obsessiv-zwanghaften Störungen bzw. Zwangsneurosen, Schizophrenie, Manien, Schlaf/Wach-Störungen, Panikattacken, Migräne, Cluster-Kopfschmerz, Schlaflosigkeit, Mischpsychose und Aufmerksamkeitsstörungen.

28. Verwendung eines alkylierenden Derivats eines Acetylakzeptorsubstrats für eine Acetyltransferase, die in der Zelle vorhanden ist, um ein Arzneimittel herzustellen, um einen Bisubstratinhibitor in einer Zelle zu erzeugen.

29. Verwendung nach Anspruch 28, wobei die Zelle eine Acetyltransferase erzeugende Zelle ist.

30. Verwendung nach Anspruch 28, wobei die Zelle Acetyltransferase aus einer exogenen Nucleinsäure erzeugt, die die Acetyltransferase codiert.

31. Verwendung nach Anspruch 28, wobei das alkylierende Derivat ausgewählt ist aus der Gruppe, die in Anspruch 4 definiert ist.

32. Verwendung nach Anspruch 28, wobei die Acetyltransferase Arylalkylamin-N-acetyltransferase (AANAT) ist und das alkylierende Derivat aus der in Anspruch 5 definierten Gruppe ausgewählt ist.

33. Verwendung nach einem der Ansprüche 28 bis 32 zur Herstellung eines Arzneimittels, um die Aktivität einer Acetyltransferase in einer Zelle zu hemmen.

34. Verwendung nach einem der Ansprüche 28 bis 32 zur Herstellung eines Arzneimittels zur Behandlung einer Störung, die durch eine verminderte Menge an Serotonin verursacht wird.

35. Verwendung nach Anspruch 28 zur Herstellung eines Arzneimittels, um die Melatoninproduktion in einer Zelle zu hemmen, die Melatonin erzeugt.

36. Verwendung nach Anspruch 28 zur Herstellung eines Arzneimittels zur Behandlung einer Störung ausgewählt aus der Gruppe bestehend aus Depression, obsessiv-zwanghaften Störungen, Schizophrenie, Manien, SchIaf/Wach-Störungen, Panikattacken, Migräne, Cluster-Kopfschmerz, Schlaflosigkeit, Mischpsychose und Aufmerksamkeitsstörungen.

## Revendications

1. Méthode de production *ex vivo* d'un inhibiteur à deux substrats dans une cellule, comprenant l'étape d'introduction dans la cellule d'un dérivé alkylant d'un substrat accepteur d'acétyle pour une acétyltransférase présente dans la cellule.

2. Méthode selon la revendication 1, dans laquelle l'acétyltransférase est produite par la cellule.

3. Méthode selon la revendication 1, dans laquelle l'acétyltransférase est produite dans une cellule par un acide nucléique exogène codant pour l'acétyltransférase.

4. Méthode selon la revendication 1, dans laquelle le dérivé alkylant du substrat accepteur d'acétyle est choisi dans le groupe consistant en un substrat accepteur d'acétyle N-bromoacétylé, un substrat accepteur d'acétyle N-chloroacétylé et un substrat accepteur d'acétyle N-fluoroacétylé.

5. Méthode selon la revendication 1, dans laquelle l'acétyltransférase est l'arylalkylamine N-acétyltransférase (AANAT) et le dérivé alkylant du substrat accepteur d'acétyle est choisi dans le groupe consistant en la N-bromoacétyltryptamine, la N-bromoacétylsérotonine, la N-bromoacétylphényléthylamine, la N-bromo-acétyl-méthoxytryptamine, la N-bromoacétyltyramine, la N-chloroacétyltryptamine, la N-chloroacétylsérotonine, la N-chloroacétylphényléthylamine, la N-chloro-acétyl-méthoxytryptamine, la N-chloroacétyltyramine, la N-fluoroacétyltryptamine, la N-fluoroacétylsérotonine, la N-fluoroacétylphényléthylamine, la N-fluoro-acétyl-méthoxytryptamine et la N-fluoroacétyltyramine.

6. Méthode d'inhibition *ex vivo* de l'activité d'une acétyltransférase dans une cellule, comprenant l'étape d'introduction dans la cellule d'un dérivé alkylant d'un substrat accepteur d'acétyle pour une acétyltransférase présente dans la cellule dans des conditions grâce auxquelles un inhibiteur à deux substrats sera produit, inhibant ainsi l'activité de l'acétyltransférase dans la cellule.

7. Méthode selon la revendication 6, dans laquelle l'acétyltransférase est produite par la cellule.

8. Méthode selon la revendication 6, dans laquelle l'acétyltransférase est produite dans une cellule par un acide nucléique exogène codant pour l'acétyltransférase.

9. Méthode selon la revendication 6, dans laquelle le dérivé alkylant du substrat accepteur d'acétyle est choisi dans le groupe consistant en un substrat accepteur d'acétyle N-bromoacétylé, un substrat accepteur d'acétyle N-chloroacétylé et un substrat accepteur d'acétyle fluoroacétylé.

10. Méthode selon la revendication 6, dans laquelle l'acétyltransférase est l'arylalkylamine N-acétyltransférase (AANAT) et le dérivé alkylant du substrat accepteur d'acétyle est choisi dans le groupe consistant en la N-bromoacétyltryptamine, la N-bromoacétylsérotonine, la N-bromoacétylphényléthylamine, la N-bromo-acétyl-méthoxytryptamine, la N-bromoacétyltyramine, la N-chloroacétyltryptamine, la N-chloroacétylsérotonine, la N-chloroacétylphényléthylamine, la N-chloro-acétyl-méthoxytryptamine, la N-chloroacétyltyramine, la N-fluoroacétyltryptamine, la N-fluoroacétylsérotonine, la N-fluoroacétylphényléthylamine, la N-fluoro-acétyl-méthoxytryptamine et la N-fluoroacétyltyramine.

11. Méthode d'inhibition *ex vivo* de la production de mélatonine dans une cellule qui produit la mélatonine, comprenant l'étape d'introduction dans la cellule d'un dérivé alkylant du substrat accepteur d'acétyle de l'AANAT qui est choisi dans le groupe consistant en la N-bromoacétyltryptamine, la N-bromoacétylsérotonine, la N-bromoacétylphényléthylamine, la N-bromo-acétyl-méthoxytryptamine, la N-bromoacétyltyramine, la N-chloroacétyltryptamine, la N-chloroacétylsérotonine, la N-chloroacétylphényléthylamine, la N-chloro-acétyl-méthoxytryptamine, la N-chloroacétyltyramine, la N-fluoroacétyltryptamine, la N-fluoroacétylsérotonine, la N-fluoroacétylphényléthylamine, la N-fluoro-acétyl-méthoxytryptamine et la N-fluoroacétyltyramine.

12. Méthode d'augmentation *ex vivo* de la quantité de sérotonine dans une cellule qui produit de la sérotonine, comprenant l'étape d'introduction dans la cellule d'un dérivé alkylant du substrat accepteur d'acétyle de l'AANAT qui est choisi dans le groupe consistant en la N-bromoacétyltryptamine, la N-bromoacétylsérotonine, la N-bromoacétylphényléthylamine, la N-bromo-acétyl-méthoxytryptamine, la N-bromoacétyltyramine, la N-chloroacétyltryptamine, la N-chloroacétylsérotonine, la N-chloroacétylphényléthylamine, la N-chloro-acétyl-méthoxytryptamine, la N-chloroacétyltyramine, la N-fluoroacétyltryptamine, la N-fluoroacétylsérotonine, la N-fluoroacétylphényléthylamine, la N-fluoro-acétyl-méthoxytryptamine et la N-fluoroacétyltyramine.

13. Cellule comprenant un inhibiteur à deux substrats, dans laquelle l'inhibiteur à deux substrats comprend un dérivé alkylant d'un substrat accepteur d'acétyle pour une acétyltransférase présente dans la cellule et CoA.

14. Cellule selon la revendication 13, dans laquelle l'acétyltransférase est produite par la cellule.

15. Cellule selon la revendication 13, dans laquelle l'acétyltransférase est produite dans la cellule par un acide nucléique exogène codant pour l'acétyltransférase.

16. Cellule selon la revendication 13, dans laquelle le dérivé alkylant du substrat accepteur d'acétyle est choisi dans le groupe consistant en un substrat accepteur d'acétyle N-bromoacétylé, un substrat accepteur d'acétyle N-chloroacétylé et un substrat accepteur d'acétyle N-fluoroacétylé.

17. Cellule selon la revendication 13, dans laquelle l'acétyltransférase est l'arylalkylamine N-acétyltransférase (AANAT) et le dérivé alkylant du substrat accepteur d'acétyle est choisi dans le groupe consistant en la N-bromoacétyltryptamine, la N-bromoacétylsérotonine, la N-bromoacétylphényléthylamine, la N-bromo-acétyl-méthoxytryptamine, la N-bromoacétyltyramine, la N-chloroacétyltryptamine, la N-chloroacétylsérotonine, la N-chloroacétylphényléthylamine, la N-chloro-acétyl-méthoxytryptamine, la N-chloroacétyltyramine, la N-fluoroacétyltryptamine, la N-fluoroacétylsérotonine, la N-fluoroacétylphényléthylamine, la N-fluoro-acétyl-méthoxytryptamine et la N-fluoroacétyltyramine.

18. Cellule selon la revendication 17, dans laquelle la cellule est choisie dans le groupe consistant en une cellule de la glande pinéale et une cellule de la rétine.

19. Dérivé alkylant d'un substrat accepteur d'acétyle pour une acétyltransférase sous la forme d'un substrat accepteur d'acétyle N-halogénoacétylé, dans lequel l'halogène est chloro, fluoro ou bromo, pour une utilisation dans un traitement médical.

20. Dérivé alkylant selon la revendication 19, qui est choisi dans le groupe consistant en la N-bromoacétyltryptamine, la N-bromoacétylsérotonine, la N-bromoacétylphényléthylamine, la N-bromo-acétyl-méthoxytryptamine, la N-bromoacétyltyramine, la N-chloroacétyltryptamine, la N-chloroacétylsérotonine, la N-chloroacétylphényléthylamine, la N-chloro-acétyl-méthoxytryptamine, la N-chloroacétyltyramine, la N-fluoroacétyltryptamine, la N-fluoroacétylsérotonine, la N-fluoroacétylphényléthylamine, la N-fluoro-acétyl-méthoxytryptamine et/ou la N-fluoroacétyltyramine.

21. Dérivé alkylant selon la revendication 19, destiné à produire un inhibiteur à deux substrats dans une cellule.

22. Dérivé alkylant selon la revendication 19, destiné à inhiber l'activité d'une acétyltransférase dans une cellule.

23. Dérivé selon l'une quelconque des revendications 19 à 22, dans lequel le dérivé est choisi dans le groupe défini dans la revendication 4.

24. Dérivé selon l'une quelconque des revendications 19 à 22, dans lequel le dérivé est un dérivé alkylant d'un substrat accepteur d'acétyle pour une arylalkylamine N-acétyltransférase et dans lequel le dérivé est choisi dans le groupe défini dans la revendication 5.

25. Dérivé alkylant selon la revendication 24, destiné à inhiber la production de mélatonine dans une cellule qui produit la mélatonine.

26. Dérivé alkylant selon la revendication 24, destiné à augmenter la quantité de sérotonine dans une cellule qui produit de la sérotonine.

27. Dérivé alkylant selon la revendication 19, pour une utilisation dans un médicament destiné à traiter un trouble choisi dans le groupe consistant en la dépression, le trouble obsessionnel compulsif, la schizophrénie, une manie ou folie, le trouble du sommeil/réveil, l'attaque de panique, la céphalée due à la migraine, la céphalée en salves, l'insomnie, la maladie bipolaire et le trouble de l'attention.

28. Utilisation d'un dérivé alkylant d'un substrat accepteur d'acétyle pour une acétyltransférase présente dans la cellule, pour préparer un médicament destiné à produire un inhibiteur à deux substrats dans une cellule.

29. Utilisation selon la revendication 28, dans laquelle la cellule est une cellule produisant l'acétyltransférase.

30. Utilisation selon la revendication 28, dans laquelle la cellule produit l'acétyltransférase par un acide nucléique exogène codant pour l'acétyltransférase.

31. Utilisation selon la revendication 28, dans laquelle le dérivé alkylant est choisi dans le groupe défini dans la revendication 4.

32. Utilisation selon la revendication 28, dans laquelle l'acétyltransférase est l'arylalkylamine N-acétyltransférase (AANAT) et le dérivé alkylant est choisi dans le groupe défini dans la revendication 5.

33. Utilisation selon l'une quelconque des revendications 28 à 32, pour préparer un médicament destiné à inhiber l'activité d'une acétyltransférase dans une cellule.

34. Utilisation selon l'une quelconque des revendications 28 à 32, pour préparer un médicament destiné à traiter un trouble provoqué par une quantité réduite de sérotonine.

35. Utilisation selon la revendication 28, pour préparer un médicament destiné à inhiber la production de mélatonine dans une cellule qui produit de la mélatonine.

36. Utilisation selon la revendication 28, pour produire un médicament destiné à traiter un trouble choisi dans le groupe consistant en la dépression, le trouble obsessionnel compulsif, la schizophrénie, une manie ou une folie, le trouble du sommeil/réveil, l'attaque de panique, la céphalée due à la migraine, la céphalée en salves, l'insomnie, la maladie bipolaire et le trouble de l'attention.
